# EUROPEAN PATENT APPLICATION

(11) **EP 4 116 315 A1**
(43) Date of publication of application: **11.01.2023**
(21) Application number: 21809787.1
(22) Date of filing: 17.05.2021
(51) Int. Cl.: C07K 1/10, C07K 1/02, C07K 14/605

(54) **METHOD FOR PREPARING LIRAGLUTIDE USING ENVIRONMENT-FRIENDLY SOLVENT**

(30) Priority: 18.05.2020 KR 20200059322
(71) Applicant: Daebong LS Co., Ltd., Namdong-gu Incheon 21697 (KR)
(72) Inventor: PARK, Eun Ju, Hwaseong-si Gyeonggi-do 18425 (KR); KIM, Eun Teak, Ansan-si Gyeonggi-do 15221 (KR); PARK, Jin Oh, Seoul 06276 (KR)
(74) Representative: Ter Meer Steinmeister & Partner
(86) International application number: PCT/KR2021/006170
(87) International publication number: WO 2021/235806

(57) **Abstract**

The present invention relates to a novel method for preparing liraglutide by means of an ionic liquid and a eutectic solvent, which are environment-friendly solvents. More specifically, the present invention is characterized in that fractionated peptide 1 represented by the following formula (1) and fractionated peptide 2 represented by the following formula (2) are subjected to a coupling reaction in the presence of an ionic liquid or a eutectic solvent. In preparing GLP-1 analogues such as liraglutide, the present invention increases reactivity when producing liraglutide, which is an unprocessed reactant, by using an ionic liquid and a eutectic solvent as environment-friendly solvents instead of using organic solvents. Accordingly, through a relatively short and simple purification process, the present invention has advantages of reducing the formation of related substances, improving purity, improving yields, shortening reaction times, reducing production cost, and lowering the manufacturing cost.

[Formula 1] (Boc-His(Trt)-Ala-Glu(tBu)-Gly-Thr[PSI(Me,Me)Pro]-Phe-Thr[PSI(Me,Me)Pro]-Ser(tBu)-Asp(tBu)-Val-Ser[PSI(Me,Me)Pro]-Ser(tBu)-Tyr(tBu)-Leu-Glu(tBu)-Gly-OH)

[Formula 2] (NH₂-Gln(Trt)-Ala-Ala-Lys(Pal-Glu-OtBu)-Glu(tBu)-Phe-Ile-Ala-Trp(Boc)-Leu-Val-Arg(Pbf)-Gly-Arg(Pbf)-Gly-linker-OH)

## Description

### [Technical Field]

The present invention relates to a novel method of preparing liraglutide using an ionic liquid and a eutectic solvent, which are eco-friendly solvents.

### [Background Art]

The peptide hormone glucagon-like peptide 1 (GLP-1) is a major polypeptide hormone with regulatory functions in glucose metabolism and gastrointestinal secretion and metabolism. GLP-1 is a growth factor for beta cells of the pancreas and has been shown to be involved in cell differentiation in organs other than the pancreas.

GLP-1 induces numerous biological effects such as stimulation of insulin secretion, inhibition of glucagon secretion, inhibition of gastric emptying, inhibition of gastric or intestinal motility, and induction of weight loss.

An important feature of GLP-1 is ability to stimulate insulin secretion without hypoglycemia-related risks associated with the use of insulin therapy or some types of oral therapy that act by increasing insulin expression. However, usefulness of therapies using GLP-1 peptides has been limited by the fact that GLP-1 is rapidly cleared *in vivo* and has a very short half-life *in vivo.*

Liraglutide (Saxenda) is an analogue of glucagon-like peptide-1 (GLP-1 (7-37)) developed by Novo Nordisk. For liraglutide, lysine at position 34 is substituted with arginine, and a palmitoyl group is bound to lysine at position 26 through a glutamoyl spacer. Thereby, liraglutide has a longer half-life than glucagon-like peptide 1 (GLP-1) and is used as an adjunct to diet and exercise to improve glycemic control in adults with type 2 diabetes.

Org. Lett., 2019, 21, 2459-2463 disclosed that a pseudo Wang linker (4-hydroxymethylphenol) is introduced to synthesize fractionated peptide 2, which is then coupled with fractionated peptide 1 that is separately synthesized, followed by removal of all protecting groups, thereby yielding crude liraglutide.

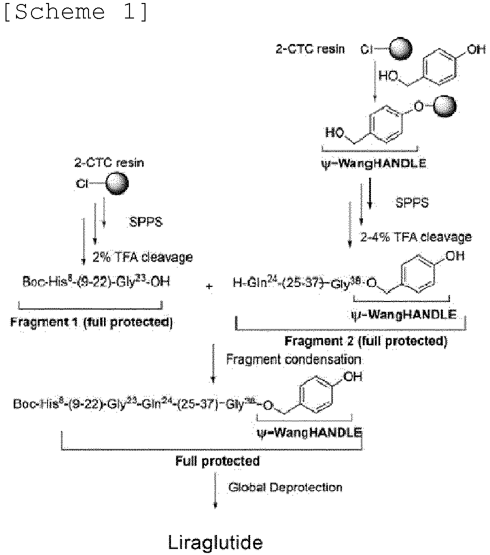

The above technique for preparing liraglutide is problematic in that the preparation time is long and the crude purity is <20%, resulting in increased preparation costs.

WO 2018/104922 A1 discloses the preparation of crude liraglutide using inorganic salts such as MgCl₂, CuCl₂, etc. in a solid-phase synthesis step.

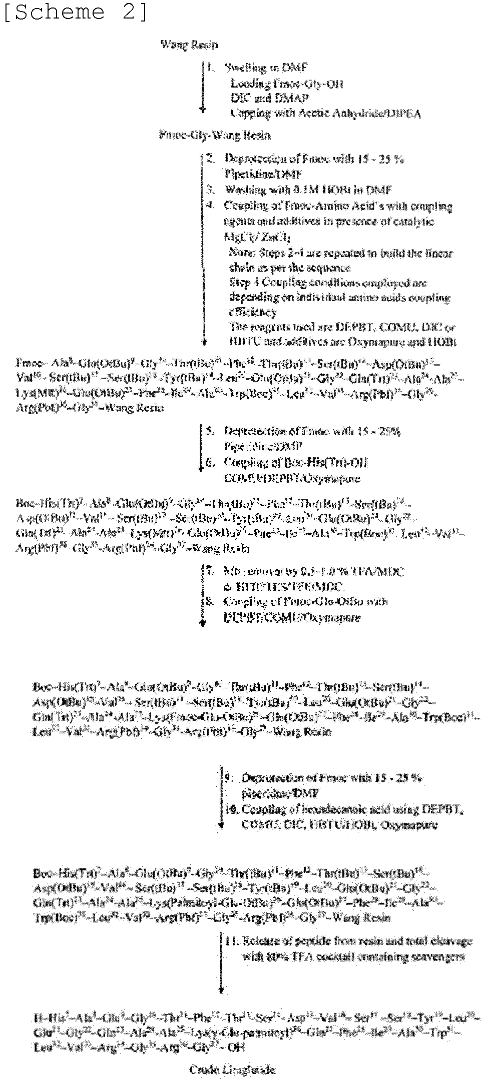

In this case, the yield of crude liraglutide is as low as 6-18%, and the purification process is performed several times using preparative HPLC and, as such, purification time and processing costs increase, which is undesirable.

In Journal of Peptide Synthesis, 2016, 22, 471-479, Pal-Glu(OSu)-OtBu is used to introduce a Pal-Glu group into the side chain of lysine, and liraglutide is prepared from fractionated peptides.

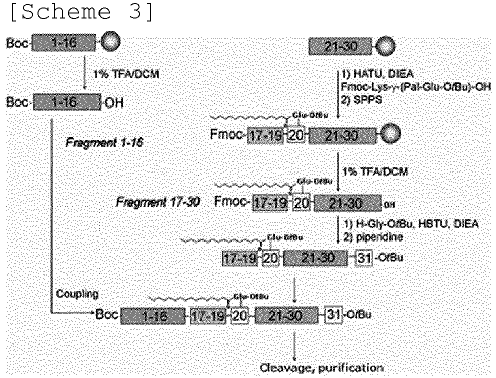

In this case, the purity of crude liraglutide is low (32.9%), and preparation costs may increase due to a long preparation time, which is undesirable.

Conventional methods for the preparation of liraglutide take a long time in solid-phase synthesis and involve a long purification process due to use of prep-HPLC, etc. for the purification of crude liraglutide. Through long processing for purification, the yield is lowered, which leads to expensive liraglutide. In conventional methods, fractionated peptides are coupled using DMSO as a solvent. Indeed, the purity of crude liraglutide obtained after the coupling reaction is very low, so a long purification process is required and the yield is also low, resulting in high costs.

Therefore, it is necessary to provide an efficient method for the preparation of liraglutide with high costeffectiveness by synthesizing crude liraglutide with high purity and high yield within a short time and making the purification process relatively short and simple.

### [Citation List]

(Patent Document 1) WO2018104922 A1 (2018.06.14.)
(Patent Document 2) WO2017162650 A1 (2017.09.28.)
(Non-Patent Document 1) Org. Lett., 2019, 21, 2459-2463
(Non-Patent Document 2) Journal of Peptide Synthesis, 2016, 22, pp. 471-479

### [Disclosure]

### [Technical Problem]

It is an object of the present invention to provide a novel method of synthesizing crude liraglutide with high purity and high yield by preparing fractionated peptides using an ultrasonic irradiation process and coupling the fractionated peptides using an ionic liquid and a eutectic solvent, which are eco-friendly solvents.

### [Technical Solution]

The present invention has been made keeping in mind the problems encountered in the related art, and provides a method of preparing liraglutide, which is capable of solving the time problem with solid-phase peptide synthesis by use of an ultrasonic irradiation process, and also which is efficient and relatively simple by use of an ionic liquid and a eutectic solvent, which are eco-friendly solvents, during coupling of fractionated peptides.

### [Advantageous effects]

According to the present invention, in the production of GLP-1 analogues such as liraglutide, when preparing liraglutide, which is a crude reaction product, an ionic liquid and a eutectic solvent are used as eco-friendly solvents instead of using an organic solvent to thus increase reactivity. By virtue of a relatively short and simple purification process, therefore, generation of productrelated substances is reduced, purity is improved, yield is increased, reaction time is shortened, and preparation and production costs are reduced.

### [Mode for Invention]

Hereinafter, a detailed description will be given of the present invention.

As used herein, "Boc" is tert-butyloxycarbonyl, "DMF" is N,N'-dimethylformamide, "Fmoc" is 9-fluorenylmethoxycarbonyl, "tBu" is tert-butyl, "TIS" is triisopropyl silane, "Trt" is trityl, "DMAP" is dimethylaminopyridine, "HOBt" is N-hydroxybenzotriazole, "TFA" is trifluoroacetic acid, "Pbf" is 2,2,4,6,7-pentamethyldihydrobenzofurane, and "Pd(PPh₃)₄" is tetrakis(triphenylphosphine)palladium.

In addition, as abbreviations of coupling agents, "PyBOP" is (benzotriazol-1-yloxy)tripyrrolidinophosphonium hexafluorophosphate, "PyAOP" is (7-azabenzotriazol-1-yloxy)tripyrrolidinophosphonium hexafluorophosphate, "PyBrop" is bromo-tris-pyrrolidino-phosphonium hexafluorophosphate, "BOP" is benzotriazol-1-yloxytris(dimethylamino)phosphonium hexafluorophosphate, "PyOxim" is [ethyl cyano(hydroxyimino)acetato-O2]tri-1-pyrrolidinylphosph onium hexafluorophosphate, "HBTU" is O-benzotriazole-N,N,N',N'-tetramethyluronium hexafluorophosphate, "HATU" is 1-[bis(dimethylamino)methylene]-1H-1,2,3-triazolo[4,5-b]pyridinium 3-oxide hexafluorophosphate, "HCTU" is O-(1H-6-chlorobenzotriazol-1-yl)-1,1,3,3-tetramethyluronium hexafluorophosphate, "TATU" is O-(7-azabenzotriazol-1-yl)-N,N,N',N'-tetramethyluronium tetrafluoroborate, "TBTU" is O-(benzotriazol-1-yl)-N,N,N',N'-tetramethyluronium tetrafluoroborate, "COMU" is (1-cyano-2-ethoxy-2-oxoethylidenaminooxy)dimethylamino-morpholino-carbenium hexafluorophosphate, "TOTT" is 2-(1-oxy-pyridin-2-yl)-1,1,3,3-tetramethylisothiouronium tetrafluoroborate, "HDMC" is N-[(5-chloro-3-oxido-1H-benzotriazol-1-yl)-4-morpholinylmethylene]-N-methylmethanaminium hexafluorophosphate, "TFFH" is fluoro-N,N,N',N'tetramethylformamidinium hexafluorophosphate, "CDI" is 1,1'-carbonyldiimidazole, "DCC" is dicyclohexylcarbodiimide, "DIC" is N,N'-diisopropylcarbodiimide, and "EDC" is 1-ethyl-3-(3-dimethylaminopropyl)carbodiimide.

In addition, if there is no special explanation or specific content, "linker" is a P. Wang linker.

The present invention pertains to a method of preparing liraglutide.

Here, fractionated peptide 1 represented by Chemical Formula 1 below and fractionated peptide 2 represented by Chemical Formula 2 below are subjected to a coupling reaction in the presence of an ionic liquid or a eutectic solvent.

[Chemical Formula 1] (Boc-His(Trt)-Ala-Glu(tBu)-Gly-Thr[PSI(Me,Me)Pro]-Phe-Thr[PSI(Me,Me)Pro]-Ser(tBu)-Asp(tBu)-Val-Ser[PSI(Me,Me)Pro]-Ser(tBu)-Tyr(tBu)-Leu-Glu(tBu)-Gly-OH)

[Chemical Formula 2] (NH₂-Gln(Trt)-Ala-Ala-Lys(Pal-Glu-OtBu)-Glu(tBu)-Phe-Ile-Ala-Trp(Boc)-Leu-Val-Arg(Pbf)-Gly-Arg(Pbf)-Gly-linker-OH)

Specifically, as shown in Scheme 4 below, fractionated peptide 1 (Fragment 1) and fractionated peptide 2 (Fragment 2) may be subjected to a coupling reaction in the presence of an ionic liquid or a eutectic solvent, and total cleavage of the coupled peptides may be carried out using TFA, phenol, TIS, or a mixture thereof, ultimately synthesizing liraglutide with high purity, high efficiency, and even more economically.

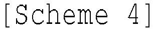

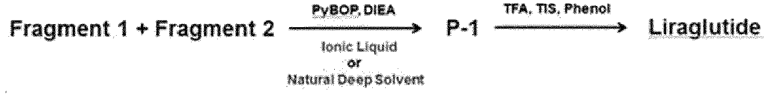

The coupling reaction of fractionated peptide 1 (Fragment 1) and fractionated peptide 2 (Fragment 2) may be carried out using at least one coupling agent selected from among PyBOP, PyAOP, PyBrop, BOP, PyOxim, HBTU, HATU, HCTU, TATU, TBTU, COMU, TOTT, HDMC, TFFH, CDI, DCC, DIC, and EDC.

Here, the coupling reaction is preferably performed under ultrasonic irradiation in order to shorten reaction time and improve productivity. Specifically, synthesis of liraglutide, having a sequence of 31 amino acids, takes a very long time, and since production time is a very important factor connected to productivity and cost reduction, higher production is possible in a short time with ultrasonic irradiation. Moreover, ultrasonic irradiation may help to improve reactivity.

The ionic liquid may be at least one selected from among 1-ethyl-3-methylimidazolium ethyl sulfate, 1-ethyl-3-methylimidazolium bromide, 1-ethyl-3-methylimidazolium hexafluorophosphate, N-butyl-N-methylpyrrolidinium bromide, N-butyl-N-methylpyrrolidinium hexafluorophosphate, N-butyl-3-methylpyridinium bromide, N-butyl-3-methylpyridinium hexafluorophosphate, tetra-N-butylammonium bromide, tetra-N-butylammonium hexafluorophosphate, tetra-N-butylphosphonium bromide, and tetra-N-butylphosphonium hexafluorophosphate, and is preferably 1-ethyl-3-methylimidazolium ethyl sulfate.

The eutectic solvent may be a mixed solvent of choline chloride and urea, a mixed solvent of choline chloride and citric acid, or a mixed solvent of citric acid and glucose, and is preferably a mixed solvent of choline chloride and urea at an equivalent ratio of 1:2.

Fractionated peptide 1 (Fragment 1) may be synthesized through a coupling reaction of peptide 1-1 represented by Chemical Formula 3 below, peptide 1-2 represented by Chemical Formula 4 below, peptide 1-3 represented by Chemical Formula 5 below, and peptide 1-4 represented by Chemical Formula 6 below.

[Chemical Formula 3] Fmoc-Tyr(tBu)-Leu-Glu(tBu)-Gly-OH

[Chemical Formula 4] Fmoc-Asp(tBu)-Val-Ser[PSI(Me,Me)Pro]-Ser(tBu)-OH

[Chemical Formula 5] Fmoc-Gly-Thr[PSI(Me,Me)Pro]-Phe-Thr[PSI(Me,Me)Pro]-Ser(tBu)-OH

[Chemical Formula 6] Boc-His(Trt)-Ala-Glu(tBu)-OH

Specifically, as shown in Scheme 5 below, respective fragments of peptides 1-1 to 1-4 are prepared, followed by a coupling reaction to obtain fractionated peptide 1 (Fragment 1). Here, it is preferable that ultrasonic irradiation be performed in order to proceed the coupling reaction efficiently.

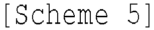

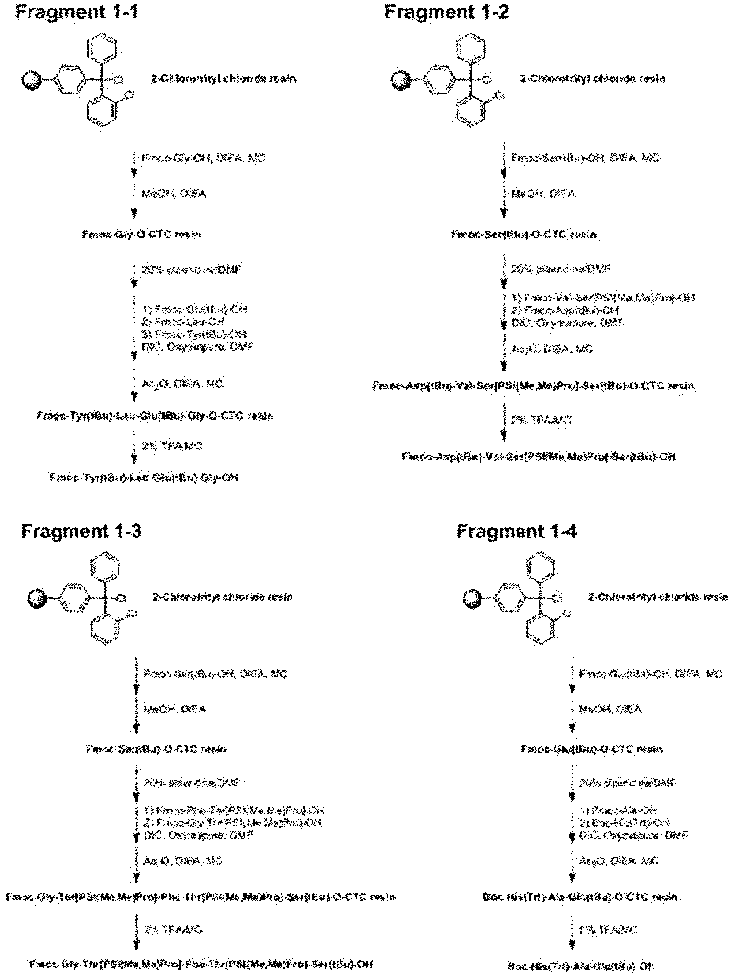

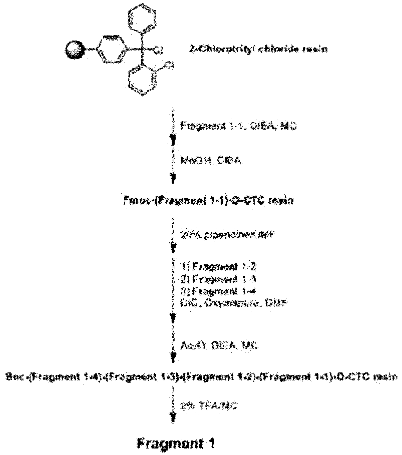

Fractionated peptide 2 (Fragment 2) may be synthesized through a coupling reaction of peptide 2-1 represented by Chemical Formula 7 below, peptide 2-2 represented by Chemical Formula 8 below, and peptide 2-3 represented by Chemical Formula 9 below.

[Chemical Formula 7] Fmoc-Val-Arg(Pbf)-Gly-Arg(Pbf)-Gly-linker-OH

[Chemical Formula 8] Fmoc-Leu-Trp(Boc)-Ala-Ile-Phe-OH

[Chemical Formula 9] Fmoc-Gln(Trt)-Ala-Ala-Lys(Pal-Glu-OtBu)-Glu(tBu)-OH

Specifically, as shown in Scheme 6 below, respective fragments of peptides 2-1 to 2-3 are prepared, followed by a coupling reaction to obtain fractionated peptide 2 (Fragment 2). Here, it is preferable that ultrasonic irradiation be performed in order to proceed the coupling reaction efficiently.

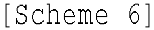

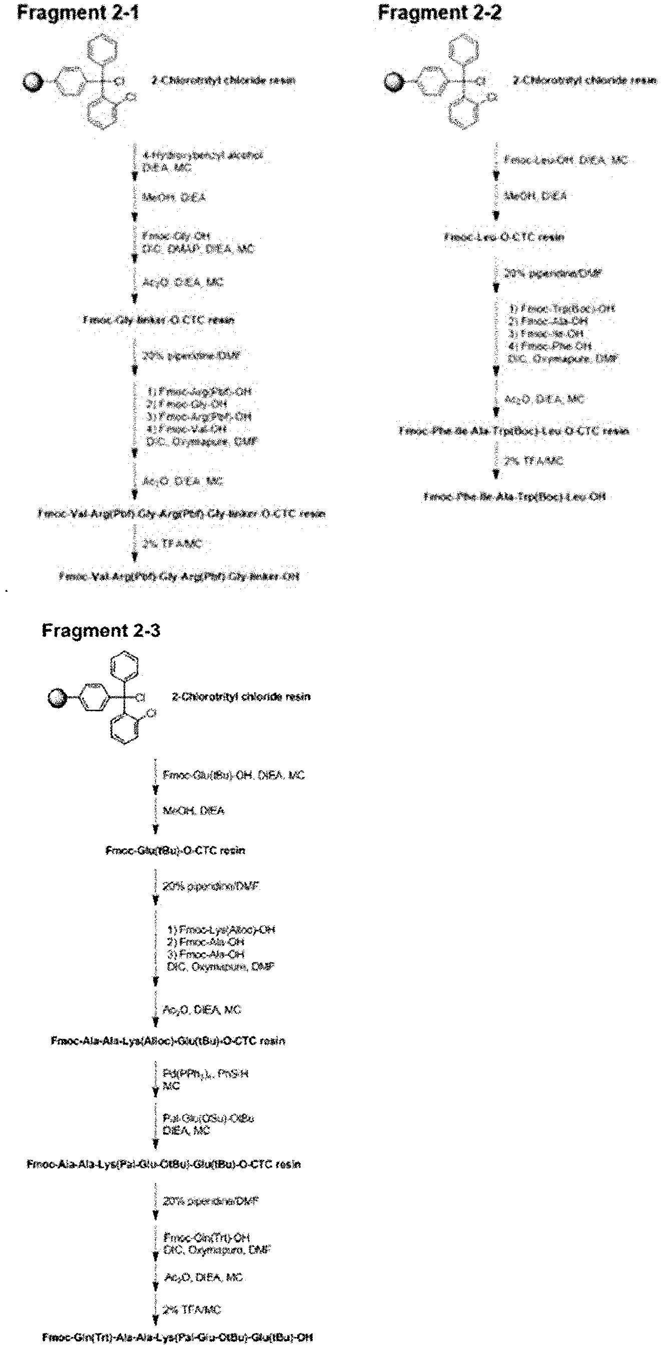

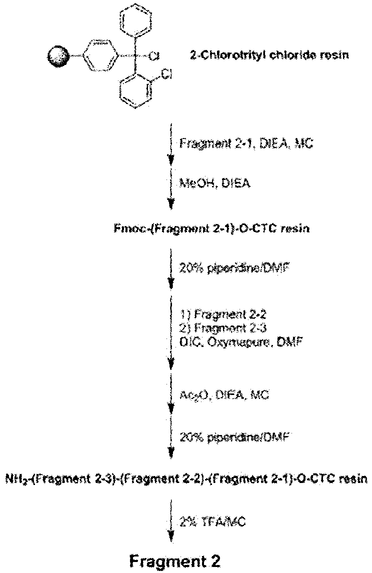

A better understating of the present invention may be obtained through the following examples. These examples are merely set forth to illustrate the present invention, and are not to be construed as limiting the scope of the present invention.

### Examples

### Example 1 - Synthesis of liraglutide using ionic liquid

### Step 1: Fractionated peptide 1-1 (Fmoc-Tyr(tBu)-Leu-Glu(tBu)-Gly-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Gly-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Glu(tBu)-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Leu-OH and Fmoc-Tyr(tBu)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v), and the resulting solution was allowed to stand at -10°C for 10 minutes. The solution was centrifuged to obtain a solid, followed by vacuum drying at room temperature for 12 hours, thereby yielding a target compound.

### Step 2: Fractionated peptide 1-2 (Fmoc-Asp(tBu)-Val-Ser[PSI(Me,Me)Pro]-Ser(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Ser(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Val-Ser[PSI(Me,Me)Pro]-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Asp(tBu)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v), and the resulting solution was allowed to stand at -10°C for 10 minutes. The solution was centrifuged to obtain a solid, followed by vacuum drying at room temperature for 12 hours, thereby yielding a target compound.

### Step 3: Fractionated peptide 1-3 (Fmoc-Gly-Thr[PSI(Me,Me)Pro]-Phe-Thr[PSI(Me,Me)Pro]-Ser(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Ser(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Phe-Thr[PSI(Me,Me)Pro]-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Gly-Thr[PSI(Me,Me)Pro]-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 4: Fractionated peptide 1-4 (Boc-His(Trt)-Ala-Glu(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Glu(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Ala-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Boc-His(Trt)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 5: Fractionated Peptide 1 (Boc-His(Trt)-Ala-Glu(tBu)-Gly-Thr[PSI(Me,Me)Pro]-Phe-Thr[PSI(Me,Me)Pro]-Ser(tBu)-Asp(tBu)-Val-Ser[PSI(Me,Me)Pro]-Ser(tBu)-Tyr(tBu)-Leu-Glu(tBu)-Gly-OH)

A 2-chlorotrityl chloride resin (1.46 mmol/g) was loaded in a peptide synthesis vessel, washed once with 10 v of MC, and allowed to swell for 30 minutes in 10 v of MC. Fractionated peptide 1-1 (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fractionated peptide 1-2 (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling fractionated peptide 1-3 and fractionated peptide 1-4. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 6: Fractionated peptide 2-1 (Fmoc-Val-Arg(Pbf)-Gly-Arg(Pbf)-Gly-linker-OH)

A 2-chlorotrityl chloride resin (1.46 mmol/g) was loaded in a peptide synthesis vessel, washed once with 10 v of MC, and allowed to swell for 30 minutes in 10 v of MC. 4-hydroxybenzyl alcohol (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-Gly-OH (3.0 eq), DIC (3.0 eq), and DMAP (0.3 eq) were dissolved in 10 v of MC and 0.1 v of DMF of the resin and placed in the peptide synthesis vessel. After stirring at room temperature for 1.5 hours, the reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted functional groups. Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Arg(Pbf)-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Gly-OH, Fmoc-Arg(Pbf)-OH, and Fmoc-Val-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 7: Fractionated Peptide 2-2 (Fmoc-Phe-Ile-Ala-Trp(Boc)-Leu-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Leu-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Trp(Boc)-OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Ala-OH, Fmoc-Ile-OH, and Fmoc-Phe-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 8: Fractionated peptide 2-3 (Fmoc-Gln(Trt)-Ala-Ala-Lys(Pal-Glu-OtBu)-Glu(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Glu(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fmoc-Lys (Alloc) -OH (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Ala-OH and Fmoc-Ala-OH. Pd(PPh₃)₄ (0.1 eq) and PhSiH (10 eq) were dissolved in MC (10 v), and the resulting solution was placed in the reactor and stirred at room temperature for 10 minutes. These procedures were repeated once more, and the resin was washed three times with MC (10 v). Pal-Glu(OSu)-OtBu (3.0 eq) was dissolved in MC (10 v), the resulting solution was placed in the peptide reactor, and DIEA (6.0 eq) was added thereto, followed by stirring at room temperature for 16 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling Fmoc-Ala-OH and Fmoc-Gln(Trt)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 9: Fractionated Peptide 2 (NH₂-Gln(Trt)-Ala-Ala-Lys(Pal-Glu-OtBu)-Glu(tBu)-Leu-Trp(Boc)-Ala-Ile-Phe-Val-Arg(Pbf)-Gly -Arg(Pbf)-Gly-linker-OH)

A 2-chlorotrityl chloride resin (1.46 mmol/g) was loaded in a peptide synthesis vessel, washed once with 10 v of MC, and allowed to swell for 30 minutes in 10 v of MC. Fractionated peptide 2-1 (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was carried out through reaction twice for 1 minute and 15 minutes using 10 v of a solution of 20% piperidine in DMF. The reaction mixture was drained and the resin was washed three times with DMF (10 v), three times with MC (10 v), and three times with DMF (10 v). Fractionated peptide 2-2 (3.0 eq), DIC (3.0 eq), and OxymaPure (3.0 eq) were mixed and then allowed to stand for 10 minutes. The solution that was allowed to stand was placed in the peptide synthesis vessel and stirred at room temperature for 1.5 hours. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection, amino-acid coupling, and capping of unreacted amine groups were repeated, thereby coupling fractionated peptide 2-3. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 10: Synthesis of liraglutide

Fractionated peptide 2 (1.0 eq) was dissolved in DMSO (12.5 v), and fractionated peptide 1 (1.1 eq) was dissolved in DMSO (12.5 v). 1-ethyl-3-methylimidazolium ethyl sulfate (25 v), 1.1 eq of (benzotriazol-1-yloxy)tripyrrolidinophosphonium hexafluorophosphate (PyBOP), and 1.1 eq of DIEA were added thereto and stirred at room temperature for 2.5 hours. A 5% sodium bicarbonate aqueous solution (100 v) was added thereto at 0-5°C, followed by centrifugation to obtain a solid. A 5% citric acid aqueous solution (100 v) was added to the solid, followed by centrifugation to obtain a solid. This solid was vacuum-dried at room temperature for 12 hours.

To the solid thus obtained, a solution of TFA, phenol, TIS, and H₂O at 88:5:5:2 (10 v) was added at 0-10°C and stirred at room temperature for 2 hours. Isopropyl ether (10 v) was added thereto at 0-5°C, followed by centrifugation to obtain a solid. Isopropyl ether (100 v) was added to the solid, followed by centrifugation to obtain a solid. This solid was vacuum-dried at room temperature for 12 hours, ultimately yielding crude liraglutide (HPLC purity: 64.5%).

### Example 2 - Synthesis of liraglutide using ionic liquid under ultrasonic irradiation

### Step 1: Fractionated Peptide 1-1 (Fmoc-Tyr(tBu)-Leu-Glu(tBu)-Gly-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Gly-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Glu(tBu)-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Leu-OH and Fmoc-Tyr(tBu)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 2: Fractionated peptide 1-2 (Fmoc-Asp(tBu)-Val-Ser[PSI(Me,Me)Pro]-Ser(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Ser(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Val-Ser[PSI(Me,Me)Pro]-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Asp(tBu)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 3: Fractionated peptide 1-3 (Fmoc-Gly-Thr[PSI(Me,Me)Pro]-Phe-Thr[PSI(Me,Me)Pro]-Ser(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Ser(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Phe-Thr[PSI(Me,Me)Pro]-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Gly-Thr[PSI(Me,Me)Pro]-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 4: Fractionated peptide 1-4 (Boc-His(Trt)-Ala-Glu(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Glu(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Ala-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Boc-His(Trt)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 5: Fractionated Peptide 1 (Boc-His(Trt)-Ala-Glu(tBu)-Gly-Thr[PSI(Me,Me)Pro]-Phe-Thr[PSI(Me,Me)Pro]-Ser(tBu)-Asp(tBu)-Val-Ser[PSI(Me,Me)Pro]-Ser(tBu)-Tyr(tBu)-Leu-Glu(tBu)-Gly-OH)

A 2-chlorotrityl chloride resin (1.46 mmol/g) was loaded in a peptide synthesis vessel, washed once with 10 v of MC, and allowed to swell for 30 minutes in 10 v of MC. Fractionated peptide 1-1 (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fractionated peptide 1-2 (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling fractionated peptide 1-3 and fractionated peptide 1-4. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 6: Fractionated peptide 2-1 (Fmoc-Val-Arg(Pbf)-Gly-Arg(Pbf)-Gly-linker-OH)

A 2-chlorotrityl chloride resin (1.46 mmol/g) was loaded in a peptide synthesis vessel, washed once with 10 v of MC, and allowed to swell for 30 minutes in 10 v of MC. 4-hydroxybenzyl alcohol (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-Gly-OH (3.0 eq), DIC (3.0 eq), and DMAP (0.3 eq) were dissolved in 10 v of MC and 0.1 v of DMF of the resin and placed in the peptide synthesis vessel. After stirring at room temperature for 1.5 hours, the reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted functional groups. Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Arg(Pbf)-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Gly-OH, Fmoc-Arg(Pbf)-OH, and Fmoc-Val-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 7: Fractionated Peptide 2-2 (Fmoc-Phe-Ile-Ala-Trp(Boc)-Leu-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Leu-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Trp(Boc)-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Ala-OH, Fmoc-Ile-OH, and Fmoc-Phe-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 8: Fractionated peptide 2-3 (Fmoc-Gln(Trt)-Ala-Ala-Lys(Pal-Glu-OtBu)-Glu(tBu)-OH)

A 2-chlorotrityl chloride resin (1.46 mmol/g) was loaded in a peptide synthesis vessel, washed once with 10 v of MC, and allowed to swell for 30 minutes in 10 v of MC. Fmoc-Glu(tBu)-OH (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fmoc-Lys(Alloc)-OH (2.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling Fmoc-Ala-OH and Fmoc-Ala-OH. Pd(PPh₃)₄ (0.1 eq) and PhSiH (10 eq) were dissolved in MC (10 v), and the resulting solution was placed in the reactor and stirred at room temperature for 10 minutes. These procedures were repeated once more, and the resin was washed three times with MC (10 v). Pal-Glu(OSu)-OtBu (3.0 eq) was dissolved in MC (10 v), the resulting solution was placed in the peptide reactor, and DIEA (6.0 eq) was added thereto, followed by ultrasonic irradiation for 30 minutes. The reaction mixture was drained and the resin was washed three times with DMF (10 v) and three times with MC (10 v). MC (10 v), acetic anhydride (1.0 eq), and DIEA (1.0 eq) were placed in the reactor and stirred at room temperature for 30 minutes, thereby capping unreacted amine groups. Fmoc-deprotection and amino-acid coupling were repeated, thereby coupling Fmoc-Ala-OH and Fmoc-Gln(Trt)-OH. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 9: Fractionated peptide 2 (NH₂-Gln(Trt)-Ala-Ala-Lys(Pal-Glu-OtBu)-Glu(tBu)-Phe-Ile-Ala-Trp(Boc)-Leu-Val-Arg(Pbf)-Gly-Arg(Pbf)-Gly-linker-OH)

A 2-chlorotrityl chloride resin (1.46 mmol/g) was loaded in a peptide synthesis vessel, washed once with 10 v of MC, and allowed to swell for 30 minutes in 10 v of MC. Fractionated peptide 2-1 (1.0 eq) was dissolved in 10 v of MC of the resin, 0.1 v of DMF and 4.0 eq of DIEA were added thereto, and the resulting mixture was placed in the peptide synthesis vessel. After stirring at room temperature for 2 hours, methyl alcohol (0.8 v) and DIEA (0.5 eq) were added to the reaction mixture and stirred at room temperature for 15 minutes. The reaction mixture was drained and the resin was washed twice with MC (10 v) and twice with DMF (10 v). Fmoc-deprotection was performed under ultrasonic irradiation for 5 minutes using 10 v of a solution of 20% piperidine in DMF. The reaction mixture was drained and the resin was washed three times with DMF (10 v), three times with MC (10 v), and three times with DMF (10 v). Fractionated peptide 2-2 (3.0 eq), HBTU (2.0 eq), HOBt (2.0 eq), and DIEA (4.0 eq) were mixed and then allowed to stand for 10 minutes. The solution that was allowed to stand was placed in the peptide synthesis vessel, followed by ultrasonic irradiation for 15 minutes. The reaction mixture was drained and the resin was washed three times with DMF (10 v) and three times with MC (10 v). Fmoc-deprotection and amino-acid coupling were repeated, thereby coupling fractionated peptide 2-3. A solution of 2% TFA in MC (10 v) was placed in the reactor, followed by reaction at room temperature for 15 minutes to obtain a reaction solution. Reaction was carried out once more, all of the reaction solution was collected, and reaction was carried out twice. The resulting reaction solution was added with isopropyl ether (100 v) and allowed to stand at -10°C for 10 minutes. The solution was centrifuged to obtain a solid, followed by vacuum drying at room temperature for 12 hours, thereby yielding a target compound.

### Step 10: Synthesis of liraglutide

Fractionated peptide 2 (1.0 eq) was dissolved in DMSO (12.5 v), and fractionated peptide 1 (1.1 eq) was dissolved in DMSO (12.5 v). 1-ethyl-3-methylimidazolium ethyl sulfate (25 v), 1.1 eq of (benzotriazol-1-yloxy)tripyrrolidinophosphonium hexafluorophosphate (PyBOP), and 1.1 eq of DIEA were added thereto and stirred at room temperature for 2.5 hours. A 5% sodium bicarbonate aqueous solution (100 v) was added thereto at 0-5°C, followed by centrifugation to obtain a solid. A 5% citric acid aqueous solution (100 v) was added to the solid, followed by centrifugation to obtain a solid. This solid was vacuum-dried at room temperature for 12 hours.

To the solid thus obtained, a solution of TFA, phenol, TIS, and H₂O at 88:5:5:2 (10 v) was added at 0-10°C and stirred at room temperature for 2 hours. Isopropyl ether (10 v) was added thereto at 0-5°C, followed by centrifugation to obtain a solid. Isopropyl ether (100 v) was added to the solid, followed by centrifugation to obtain a solid. This solid was vacuum-dried at room temperature for 12 hours, ultimately yielding crude liraglutide (HPLC purity: 65.5%).

### Example 3 - Synthesis of liraglutide using eutectic solvent

### Steps 1 to 9

These steps were performed in the same manner as in Example 2.

### Step 10: Synthesis of liraglutide

Fractionated peptide 2 (1.0 eq) was dissolved in DMSO (12.5 v), and fractionated peptide 1 (1.1 eq) was dissolved in DMSO (12.5 v). A mixed solvent (25 v) of choline chloride and urea at a ratio of 1:2, 1.1 eq of (benzotriazol-1-yloxy)tripyrrolidinophosphonium hexafluorophosphate (PyBOP), and 1.1 eq of DIEA were added thereto and stirred at room temperature for 2.5 hours. A 5% sodium bicarbonate aqueous solution (100 v) was added thereto at 0-5°C, followed by centrifugation to obtain a solid. A 5% citric acid aqueous solution (100 v) was added to the solid, followed by centrifugation to obtain a solid. This solid was vacuum-dried at room temperature for 12 hours.

To the solid thus obtained, a solution of TFA, phenol, TIS, and H₂O at 88:5:5:2 (10 v) was added at 0-10°C and stirred at room temperature for 2 hours. Isopropyl ether (10 v) was added thereto at 0-5°C, followed by centrifugation to obtain a solid. Isopropyl ether (100 v) was added to the solid, followed by centrifugation to obtain a solid. This solid was vacuum-dried at room temperature for 12 hours, ultimately yielding crude liraglutide (HPLC purity: 67.5%).

Compared to conventional methods, particularly solidor liquid-phase preparation methods using an organic solvent disclosed in Patent Documents 1 and 2 and Non-Patent Documents 1 to 3, the present invention exhibits improved effects in the overall process, such as remarkably short preparation time, high purity, and high yield.

Specifically, the conventional methods may cause problems in processing or environment owing to the use of an organic solvent, and the purity is about 10-20%, the yield is about 5-15%, and the total preparation time is about 15-20 days.

In contrast, the present invention obviates the need to use an organic solvent, so there is no fear of causing problems in processing or environment, the purity is 65% or more, the yield is 30% or more, and the total preparation time is shortened to about 3-4 days.

Moreover, compared to the conventional methods, the novel method of the present invention uses inexpensive materials or reagents in processes and is thus highly competitive in view of preparation costs.

Consequently, the present invention is capable of producing liraglutide very economically when applied to a mass production process based on such improved effects.

## Claims

1. A method of preparing liraglutide, comprising subjecting fractionated peptide 1 represented by Chemical Formula 1 below and fractionated peptide 2 represented by Chemical Formula 2 below to a coupling reaction using an ionic liquid or a eutectic solvent.
[Chemical Formula 1] (Boc-His(Trt)-Ala-Glu(tBu)-Gly-Thr[PSI(Me,Me)Pro]-Phe-Thr[PSI(Me,Me)Pro]-Ser(tBu)-Asp(tBu)-Val-Ser[PSI(Me,Me)Pro]-Ser(tBu)-Tyr(tBu)-Leu-Glu(tBu)-Gly-OH)
[Chemical Formula 2] (NH₂-Gln(Trt)-Ala-Ala-Lys(Pal-Glu-OtBu)-Glu(tBu)-Phe-Ile-Ala-Trp(Boc)-Leu-Val-Arg(Pbf)-Gly-Arg(Pbf)-Gly-linker-OH)

2. The method according to claim 1, wherein the ionic liquid is at least one selected from among 1-ethyl-3-methylimidazolium ethyl sulfate, 1-ethyl-3-methylimidazolium bromide, 1-ethyl-3-methylimidazolium hexafluorophosphate, N-butyl-N-methylpyrrolidinium bromide, N-butyl-N-methylpyrrolidinium hexafluorophosphate, N-butyl-3-methylpyridinium bromide, N-butyl-3-methylpyridinium hexafluorophosphate, tetra-N-butylammonium bromide, tetra-N-butylammonium hexafluorophosphate, tetra-N-butylphosphonium bromide, and tetra-N-butylphosphonium hexafluorophosphate.

3. The method according to claim 1, wherein the eutectic solvent is a mixed solvent of choline chloride and urea, a mixed solvent of choline chloride and citric acid, or a mixed solvent of citric acid and glucose.

4. The method according to claim 3, wherein the eutectic solvent is a mixed solvent of choline chloride and urea at an equivalent ratio of 1:2.

5. The method according to claim 1, wherein the fractionated peptide 1 is synthesized through a coupling reaction of peptide 1-1 represented by Chemical Formula 3 below, peptide 1-2 represented by Chemical Formula 4 below, peptide 1-3 represented by Chemical Formula 5 below, and peptide 1-4 represented by Chemical Formula 6 below.
[Chemical Formula 3] Fmoc-Tyr(tBu)-Leu-Glu(tBu)-Gly-OH
[Chemical Formula 4] Fmoc-Asp(tBu)-Val-Ser[PSI(Me,Me)Pro]-Ser(tBu)-OH
[Chemical Formula 5] Fmoc-Gly-Thr[PSI(Me,Me)Pro]-Phe-Thr[PSI(Me,Me)Pro]-Ser(tBu)-OH
[Chemical Formula 6] Boc-His(Trt)-Ala-Glu(tBu)-OH

6. The method according to claim 1, wherein the fractionated peptide 2 is synthesized through a coupling reaction of peptide 2-1 represented by Chemical Formula 7 below, peptide 2-2 represented by Chemical Formula 8 below, and peptide 2-3 represented by Chemical Formula 9 below.
[Chemical Formula 7] Fmoc-Val-Arg(Pbf)-Gly-Arg(Pbf)-Gly-linker-OH
[Chemical Formula 8] Fmoc-Phe-Ile-Ala-Trp(Boc)-Leu-OH
[Chemical Formula 9] Fmoc-Gln(Trt)-Ala-Ala-Lys(Pal-Glu-OtBu)-Glu(tBu)-OH

7. The method according to claim 1, wherein the coupling reaction of fractionated peptide 1 and fractionated peptide 2 is performed using at least one coupling agent selected from among PyBOP, PyAOP, PyBrop, BOP, PyOxim, HBTU, HATU, HCTU, TATU, TBTU, COMU, TOTT, HDMC, TFFH, CDI, DCC, DIC, and EDC.

8. The method according to any one of claims 1 to 7, wherein the coupling reaction is performed under ultrasonic irradiation.
